Europäisches Patentamt

European Patent Office

Office européen des brevets

(11) Publication number: **0 077 016**
**B1**

(12) **EUROPEAN PATENT SPECIFICATION**

(45) Date of publication of patent specification: **19.06.85**

(51) Int. Cl.⁴: $C\ 07\ C\ 85/18$, $C\ 07\ C\ 87/10$

(21) Application number: **82109235.0**

(22) Date of filing: **06.10.82**

(54) Amines via the amination of olefins using select zeolites.

(30) Priority: **07.10.81 US 309338**

(43) Date of publication of application:
**20.04.83 Bulletin 83/16**

(45) Publication of the grant of the patent:
**19.06.85 Bulletin 85/25**

(84) Designated Contracting States:
**AT BE CH DE FR GB IT LI LU NL SE**

(56) References cited:
**EP-A-0 039 918**
**DE-A-1 470 650**

**J.A. RABO: "ZEOLITE CHEMISTRY AND
CATALYSIS", 1976, American Chemical
Society, Washington (US)**

(73) Proprietor: **AIR PRODUCTS AND CHEMICALS,
INC.**
**P.O. Box 538**
**Allentown, Pennsylvania 18105 (US)**

(72) Inventor: **Deeba, Michel**
**1080G Coldstream Circle**
**Emmaus, PA, 18049 (US)**
Inventor: **Ambs, William J.**
**325 Dartmouth Avenue**
**Swarthmore, PA 19081 (US)**

(74) Representative: **Berg, Wilhelm, Dr. et al
Patentanwälte Dr. Berg Dipl.-Ing. Stapf Dipl.-Ing.
Schwabe Dr. Dr. Sandmair Postfach 86 02 45
Stuntzstrasse 16
D-8000 München 86 (DE)**

Courier Press, Leamington Spa, England.

## Description

Technical field
This invention relates to a process for producing amines by the reaction of ammonia or ammonia type compound with an olefin.

Background of the prior art
The earliest work relating to the manufacture of amines by the amination of olefins particularly ethylamines by the amination of ethylene, appears to have been done by Teter et al as noted in U.S. Patents 2,623,061; 2,479,879; 2,417,892; 2,381,470; 2,658,041; 2,381,709; 2,392,107 and 2,398,899. These patents show that ammonia can be made to react with olefins, e.g. ethylene to produce amines. As noted by others through improvements in the process, substantial quantities of polyamines and polyolefins were produced by the Teter et al catalyst which is a metal supported on a spinel type support, silica and diatomaceous earth and the like.

*Olin et al* in U.S. Patents 2,422,631 and 2,422,632, discloses a process for producing amines and amides by the reaction of a monounsaturated olefin, carbon monoxide and an amine or ammonia. The catalyst used is a combination of a dehydrating and a hydrogenation catalyst, e.g. nickel and activated alumina, copper and silica gel, etc.

*Whitman*, U.S. 2,501,556 discloses a liquid phase reaction of ammonia and ethylene in the presence of an alkali metal catalyst to form ethylamine.

*McClain*, U.S. 3,412,158 discloses a gas phase process for producing primary alkyl amines from low molecular weight olefins and ammonia by carrying out the gas phase process in the presence of a noble metal containing catalyst at temperatures of from 90—175°C, at pressures of from atmospheric to $1.4 \times 10^7$Pa (2,000 psig).

EP—A—0 039 918 describes a vapor phase process for the production of amines by reaction of $C_{2-8}$ olefins with ammonia or amines, the process comprising the application of an alumino silicate as catalyst. This process offers only a moderate conversion of olefins to amines.

DE—A—1 470 650 describes a process and a catalyst composition for the conversion of hydrocarbons. The possible use of a modified clinoptilotite in the amination of olefins is mentioned therein, but no example is provided.

It is also known that alumino-silicates and particularly zeolites can be used for effecting amination of olefins at temperatures of 200—500°C, pressures from $2.1 \times 10^6$—$4.1 \times 10^7$Pa (300—6,000 psig). High yields and selectivity are reported.

Summary of the invention
This invention relates to an improved process for the preparation of ethylamines by the vapor phase catalytic reaction of ethylene and ammonia. In the basic process ethylene is reacted at a temperature and pressure sufficient to effect formation of the amine, but controlled to prevent polymer formation. The improvement generally resides in the use of
(a) a temperature from 300—400°C,
(b) a pressure of from $2.1 \times 10^6$—$1.4 \times 10^7$Pa (300—2,000 psig), and
(c) an acidic alumino-silicate selected from the group consisting of erionite, clinoptilolite and mixtures.
There are several advantages either singly or combination associated with the invention as compared to many prior art processes. These advantages include:
an ability to produce ethylamines in high selectivity without producing substantial by-products (greater than 8%), e.g. polymers in the form of polyolefins or hydrocarbons, e.g., linear, branched, or cyclic. The production of polymers has been a serious problem with many of the prior art processes;
an ability to use a gas phase reaction which permits high production rate in producing amines;
an ability to reduce the amount of by-product in the form of by-product nitrogen compounds e.g. nitriles as compared to prior art processes;
an ability to obtain good to high conversion of ethylene to amine by virtue of the use of the particular catalyst and conditions employed and good catalyst life; and
an ability to obtain a low aging rate with the particular catalysts.

Description of the preferred embodiment
In the amination process ethylene is reacted with ammonia to produce the amine. Higher molecular weight olefins, e.g. propylene and butylene are more difficult to aminate with these catalysts and have extremely lower conversion. Presumably this is due to the small pore size of the catalyst and the large size of the higher amine molecules. Ammonia type reactants, e.g. primary and secondary amines also result in low conversion, presumably for the same reasons as the higher olefins.

In the process, ammonia is reacted with ethylene at temperatures broadly from 300—400°C. Lower temperatures result in lower conversion and higher temperatures result in lower selectivity which generally appears in the form of a nitrile or hydrocarbon.

Pressures suited for practicing the invention are from $2.1 \times 10^6$—$1.4 \times 10^7$Pa (300—2,000 psig) with preferred pressures of from $4.1 \times 10^6$—$7.6 \times 10^6$Pa (600—1100 psig). Generally, pressures lower than

2

$4.1\times10^6$Pa (600 psig) result in poor conversion of ethylene to amine. On the other hand, as the pressure is increased above $1.03\times10^7$Pa (1500 psig) conversion is increased only slightly. In order to minimize the recovery problems associated with separating the amine from the reactants and other by-products, higher conversions can be waived and pressures of from $4.1\times10^6$—$7.6\times10^6$Pa used as this permits extremely high selectivities, e.g. greater than 95%.

Another important variable in the gas phase amination of the ethylene is the mole ratio of ammonia to ethylene in the feed. Generally, the mole ratio can be from about 0.2—20 moles ammonia per mole of ethylene with preferred ranges being from about 1—10 to 1 ammonia to ethylene. Mole ratios higher than about 10:1 of course require greater energy expenditure to handle the larger quantities of gas that pass through the reactor, and there seems to be no significant advantage in terms of increased selectivity or conversion at these higher ratios of ammonia to ethylene. On the other hand, as the mole ratio of ammonia to ethylene falls below about 0.2, there is a loss of selectivity to the amine.

The gas hourly space velocity (GHSV), which has units of volume of gas per volume of catalyst in unit time i.e. (cc gas at STP)/(cc catalyst hours$^{-1}$), can be from 500—5,000 with a preferred range being from 750—2,000. As the space velocity is increased toward the upper end of the range, conversion generally falls dramatically, particularly above 3,000. On the other hand, as the space velocity approaches the lower level of 500, selectivity decreases and by-products form in greater concentration.

One of the important factors in achieving the results of high selectivity to amines without the concomitant production of substantial amounts of by products in the form of polymer, e.g. polyethylene; hydrocarbon or nitrogen compound as encountered in the prior art processes including those using zeolites is in the use of a highly acidic alumino-silicate selected from the group consisting of erionite, clinoptilolite and mixtures thereof. These zeolites are highly selective for the amination of ethylene and produce unique results. For example chabazite, which is a natural zeolite, gives lower conversion and selectivity even when used in combination with these zeolites. Thus, when using these catalysts at least about 80% by weight and preferably all of the catalyst is erionite, clinoptilolite or mixtures. It is believed the high acidity and narrow pore size of these alumino-silicates plays an important role in the catalyst activity and selectivity. Erionite and clinoptilolite are generally naturally occurring.

Various ions can be incorporated into the zeolite by conventional techniques to provide acidity. Trivalent ions, and particularly the rare earth metals, i.e. lanthanum, neodymium, praseodymium, are well suited for preparing exchanged zeolites. Other cations which may be used in place of the original metal cations of the erionite or clinoptilolite include hydrogen, barium, beryllium, calcium, cerium, magnesium, potassium, silver and zinc. However, sodium and potassium tend to reduce activity presumably due to the lowering of the acidity of the catalyst.

The crystalline alumino-silicate of the present invention may be employed alone or in combination with various binder or matrix materials (organic or inorganic materials, resins, clays, gels or the like). The physical form of the catalyst may be adapted to use in any conventional catalytic system (e.g. slurry or fluidized bed systems, beads, pellets or the like for use in fixed or moving bed catalysts). As is known, the hydrogen form of the zeolite can be prepared from the ammonium form and for purposes herein is referred to as the hydrogen form. The preferred metal ions for the exchanged zeolites are lanthanum and hydrogen as these provide good yields, good selectivity and good catalyst life.

The following examples are provided to illustrate preferred embodiments of the invention and are not intended to restrict the scope thereof.

Example 1

A series of amination reactions 1—36 and 43—63 were carried out using a reactor consisting of a stainless steel tubing 22.86 cm (9 inches) long of 1.9 cm (3/4'') outside diameter and 0.8 cm (5/16'') inside diameter. The stainless steel reactor was mounted inside a close fitting block of Inconel metal which was instrumented for temperature control. A thermowell extending axially through the reactor was used to measure reactor temperature.

The catalyst volume for the reactor was approximately 6 cubic centimeters and the catalyst was held in place by a quartz wool plug. The catalysts were sieved to pass through a 12 U.S. standard sieve but retained on an 18 mesh sieve.

In the runs the experimental catalysts used were prepared from a 50—50 (as sold commercially by Anaconda Corp.) mixture of chabazite and erionite and from erionite and clinoptilolite separately, and SK 500. Such catalysts were prepared in the H form by conventional exchange of the naturally occurring zeolite followed by forming into pellets and heat treating. Comparisons were made against Linde SK-500 zeolite (REY — a rare earth exchanged zeolite). A typical example of such catalyst preparation is as follows:

A 300 g sample of clinoptilolite was ammonium exchanged, then mixed with water to form an extrudable mix. This mixture then was thoroughly blended to assure homogeneity. After blending, the product was extruded into 0.32 cm (1/8 inch) pellets and dried. The dried product was heated at 800°F for 2 hours then crushed and sieved.

Table 1 represents results for various catalysts including natural zeolites and reactor conditions. Table 2 is an average summary of work carried out in the manner of Table 1. MEA and DEA represent mono and diethylamine respectively.

3

TABLE 1
Amination of ethylene

| Run | Olefin | Catalyst | T, °C | $10^6$Pa | Psig | GHSV | NH$_3$/Ethylene mole ratio | % Conv. | % Selectivity | | |
|---|---|---|---|---|---|---|---|---|---|---|---|
| | | | | | | | | | Mono amines | Diamine | Other |
| 1 | Ethylene | H Chabazite Erionite 50-50 | 335 | 5.27 | 765 | 2000 | 4.1 | 1.9 | 100 | | |
| 2 | " | " | 350 | 5.27 | 765 | 2000 | 4.1 | 4.7 | 100 | | |
| 3 | " | " | 368 | 5.27 | 765 | 2000 | 4.1 | 7.0 | 100 | | |
| 4 | " | " | 370 | 5.27 | 765 | 2000 | 4.1 | 7.22 | 99 | 1 | |
| 5 | " | " | 385 | 5.27 | 765 | 2000 | 4.1 | 7.0 | 98 | 2 | |
| 6 | " | " | 340 | 5.17 | 750 | 2000 | 4.0 | 4.12 | 100 | — | |
| 7 | " | " | 360 | 5.17 | 750 | 2000 | 4.0 | 9.8 | 80.5 | — | |
| 8 | " | " | 370 | 5.17 | 750 | 2000 | 3.7 | 10.6 | 93 | 6 | 1 |
| 9 | " | " | 370 | 5.17 | 750 | 2000 | 4.0 | 11.1 | 94 | 6 | |
| 10 | " | " | 380 | 5.17 | 750 | 2000 | 3.6 | 12.3 | 91 | 7.7 | 1.3 |
| 11 | " | H chabazite-erionite macroporous | 320 | 5.17 | 750 | 2000 | 4.2 | 1.9 | 100 | — | |
| 12 | " | " | 350 | 5.17 | 750 | 2000 | 4.0 | 5.3 | 100 | — | |
| 13 | " | " | 370 | 5.17 | 750 | 2000 | 4.2 | 10.5 | 95 | 5 | |
| 14 | " | " | 380 | 5.17 | 750 | 2000 | 4.3 | 11.3 | 94.4 | 5.6 | |
| 15 | " | " | 390 | 5.17 | 750 | 2000 | 4.4 | 12.6 | 82 | 4.1 | 13.9 |

0 077 016

TABLE 1 (contd.)
Amination of ethylene

| Run | Olefin | Catalyst | T, °C | $10^6$Pa | Psig | GHSV | $NH_3$/Ethylene mole ratio | % Conv. | % Selectivity Mono amines | Diamine | Other |
|---|---|---|---|---|---|---|---|---|---|---|---|
| 16 | " | Clinoptilolite | 340 | 5.17 | 750 | 2000 | 4.5 | 4.4 | 100 | — | — |
| 17 | " | " | 340 | 5.17 | 750 | 2000 | 4.9 | 6.6 | 100 | — | — |
| 18 | " | " | 360 | 5.17 | 750 | 2000 | 4.6 | 11.4 | 100 | — | — |
| 19 | " | " | 370 | 5.17 | 750 | 2000 | 4.4 | 13.7 | 93.1 | 6.9 | — |
| 20 | " | " | 370 | 5.17 | 750 | 2000 | 4.6 | 14.9 | 91.4 | 8.6 | — |
| 21 | " | " | 380 | 5.17 | 750 | 2000 | 4.4 | 14.8 | 91.9 | 8.1 | — |
| 22 | " | " | 380 | 5.17 | 750 | 2000 | 4.7 | 14.4 | 92 | 8 | — |
| 23 | " | " | 390 | 5.17 | 750 | 2000 | 4.4 | 15.7 | 91 | 9 | — |
| 24 | " | " | 400 | 5.17 | 750 | 2000 | 4.7 | 18 | 90.2 | 9.8 | — |
| 25 | " | SK-500 | 350 | 5.17 | 750 | 2000 | 4.6 | 3.9 | 100 | — | — |
| 26 | " | " | 360 | 5.17 | 750 | 2000 | 4.3 | 7.5 | 100 | — | — |
| 27 | " | " | 370 | 5.17 | 750 | 2000 | 4.3 | 9.4 | 96 | 4 | — |
| 28 | " | " | 380 | 5.17 | 750 | 2000 | 4.2 | 12.1 | 92.6 | 5.4 | — |
| 29 | " | " | 390 | 5.17 | 750 | 2000 | 4.0 | 12.8 | 81.9 | 4.5 | 13.6 |

TABLE 1 (contd.)
Amination of ethylene

| Run | Olefin | Catalyst | T, °C | 10⁶Pa | Psig | GHSV | NH₃/Ethylene mole ratio | % Conv. | % Selectivity | | |
|-----|--------|----------|-------|-------|------|------|-------------------------|---------|---------------|---|---|
| | | | | | | | | | Mono amines | Diamine | Other |
| 30 | " | SK-500 | 400 | 5.17 | 750 | 2000 | 3.8 | 13 | 69 | 2.0 | 28.9 |
| 31 | " | " | 320 | 5.17 | 750 | 2000 | 4.0 | — | — | | |
| 32 | " | " | 335 | 5.17 | 750 | 2000 | 4.0 | 2.5 | 100 | | |
| 33 | " | " | 335 | 5.17 | 750 | 2000 | 3.8 | 2.9 | 100 | | |
| 34 | " | " | 355 | 5.17 | 750 | 2000 | 4.3 | 3.4 | 100 | | |
| 35 | " | " | 360 | 5.17 | 750 | 2000 | 3.8 | 8.8 | 97 | | 3.0 |
| 36 | " | " | 370 | 5.17 | 750 | 2000 | 4.1 | 12.3 | 90 | 7.3 | 2.7 |
| 43 | " | H Erionite | 360 | 5.17 | 750 | 2000 | 3.4 | 9.3 | 88.5 | 10.7 | 0.8 |
| 44 | " | " | 375 | 5.17 | 750 | 2000 | 8.0 | 15.5 | 88.4 | 10.5 | 1.1 |
| 45 | " | " | 380 | 5.17 | 750 | 2000 | 3.5 | 12.3 | 84.7 | 13.8 | 1.5 |
| 46 | " | " | 380 | 5.17 | 750 | 2000 | 5.2 | 9.9 | 88.5 | 8.8 | 2.7 |
| 47 | " | " | 380 | 5.17 | 750 | 2000 | 4.1 | 13.5 | 83.3 | 12.3 | 2.6 |
| 48 | " | " | 380 | 5.17 | 750 | 2000 | 4.7 | 12.9 | 87.8 | 10.0 | 2.2 |
| 49 | " | " | 380 | 5.17 | 750 | 2000 | 5.5 | 13.0 | 88.4 | 8.7 | 2.9 |

TABLE 1 (contd.)
Amination of ethylene

| Run | Olefin | Catalyst | T, °C | $10^6$Pa | Psig | GHSV | NH₃/Ethylene mole ratio | % Conv. | % Selectivity Mono amines | Diamine | Other |
|-----|--------|----------|-------|----------|------|------|------|---------|-------------|---------|-------|
| 50 | " | H Erionite | 380 | 5.17 | 750 | 2000 | 5.6 | 14.4 | 87 | 8.5 | 4.5 |
| 51 | " | " | 390 | 5.17 | 750 | 2000 | 5.8 | 15.1 | 86.2 | 8.4 | 5.0 |
| 52 | " | " | 390 | 5.17 | 750 | 2000 | 5.8 | 16.3 | 84.3 | 8.6 | 5.6 |
| 53 | " | " | 400 | 5.17 | 750 | 2000 | 5.7 | 17.5 | 77.7 | 7.7 | 14.6 |
| 54 | " | " | 400 | 5.17 | 750 | 2000 | 5.8 | 17.9 | 78.6 | 7.9 | 13.2 |
| 55 | " | " | 320 | 5.17 | 750 | 2000 | 3.8 | 1.6 | 90.8 | 3.7 | 5.5 |
| 56 | " | " | 320 | 5.17 | 750 | 2000 | 4.3 | 2.8 | 95.4 | 4.5 | — |
| 57 | " | " | 340 | 5.17 | 750 | 2000 | 4.7 | 3.5 | 97.2 | 2.7 | |
| 58 | " | " | 360 | 5.17 | 750 | 2000 | 4.5 | 7.4 | 92.4 | 6.7 | 0.8 |
| 59 | " | " | 360 | 5.17 | 750 | 2000 | 3.0 | 8.7 | 88.6 | 10.5 | 1.0 |
| 60 | " | " | 370 | 5.17 | 750 | 2000 | 2.7 | 10.2 | 86.2 | 12.5 | 1.2 |
| 61 | " | " | 380 | 5.17 | 750 | 2000 | 3.2 | 13.4 | 85.4 | 11.7 | 1.7 |
| 62 | " | " | 380 | 5.17 | 750 | 2000 | 4.0 | 15.2 | 85.7 | 12.5 | 2.3 |
| 63 | " | " | 400 | 5.17 | 750 | 2000 | 3.9 | 16.5 | 84.2 | 13.1 | 2.5 |

In Runs 1—36 correction factors for GC calibration have not been applied. When applied, % conversions will decrease about 15% from that stated. In Runs 43—63 a correction factor of 0.6 for MEA and 0.7 for C₂H₄ was introduced.

### TABLE 2
Comparison of ethylene conversion and amine selectivity as a function of temperature

| Temp. | SK-500* | | H-clinoptilolite | | H-erionite | |
|---|---|---|---|---|---|---|
| | A | B | A | B | A | B |
| 320 | — | — | 2.2 | 100 | 2.9 | 100 |
| 340 | 1.5 | 100 | 5.6 | 100 | 5.26 | 100 |
| 350 | 3.3 | 100 | | | | |
| 360 | 5.3 | 100 | 9.7 | 100 | 8.76 | 99.2 |
| 370 | 8.0 | 100 | 11.7 | 100 | 12.52 | 98.7 |
| 380 | 10.3 | 98 | 12.6 | 100 | 15.2 | 97.3 |
| 390 | 10.8 | 86.4 | 14.6 | 100 | | |
| 400 | 11.0 | 71 | 15.3 | 99.6 | 16.9 | 94 |

$*$ = % $C_2H_4$ conversion was corrected by introducing correction factors of 0.7 and 0.6 for $C_2H_4$ and MEA respectively.
A = % ethylene conversion.
B = selectivity to ethylamines.

These summarizing data show that the H-clinoptilolite and H erionite consistently give better selectivity and better conversion than SK-500 at a given temperature and pressure, mole ratio, etc. The data in Table 1 show better conversion for these catalysts than with the chabazite mixtures even when "uncorrected" in runs 1—36.

From the data in the tables it is clear that the erionite and clinoptilolite catalysts are extremely effective for the amination of ethylene and they were more effective than the standard Linde SK-500 zeolite (a rare earth exchanged Y zeolite), and chabazite mixtures. Conversions were significantly higher particularly with the H-clinoptilolite and H-erionite catalyst at temperatures from about 370—400°C. In those cases where conversions were forced with SK-500, the corresponding selectivity to the desired amine products was lower than where higher conversions were achieved using clinoptilolite and erionite, note runs 19—30 and Table 2. Another interesting point about H clinoptilolite and H erionite is that only a small fraction of the product was non-amine even at high conversions whereas higher by-product levels were experienced with SK-500. Runs 29, 30 and 61—63 show this point.

The higher catalytic activity of H-clinoptilolite and erionite as noted above was attributed to the higher acidity of the catalyst. To confirm this belief, acidity distribution was measured. A thermal gravimetric analysis technique with ammonia as an adsorbate was used. The acidity measurement being performed by activating about 20—40 mg of catalyst at temperatures up to 400°C in helium, after which the catalyst was cooled at 25°C. The uptake of ammonia by the catalysts was very fast and the catalyst surface saturated within 5 minutes. Helium was then used to desorb the physically adsorbed ammonia at 25°C followed by heating the catalyst to 100, 200, 300, and 400°C respectively to desorb physically adsorbed ammonia. The temperature was taken up after there is no change in the rate of desorption (as noted by decreasing weight). The amount of irreversibly adsorbed ammonia at each temperature was taken as a count of acid sites. The amount of irreversibly adsorbed ammonia at 25 and 100°C was considered as a count of total acidity (weak plus strong acid sites) and that at 200 and 300°C as strong acidic sites. The strong acidic sites were considered as the number of surface active sites for ethylene amination. Table 3 shows a comparison of acidic site distribution over SK-500, H-clinotilolite, and H-erionite. The acidity of these materials decreases in the order H-erionite, H-clinoptilolite and SK-500. The same trend was observed for the amination activity. The enhanced acidity of H-erionite and H-clinoptilolite over SK-500 also would explain the superior activity of the catalysts, while the better selectivity may be related to the pore shape structure.

TABLE III
The number of acid site distribution

(mmoles of ammonia/g of dry catalyst)

| Catalyst | Temp °C | | | |
|---|---|---|---|---|
| | 25 | 100 | 200 | 300 |
| SK-500 | 3.3 | 1.8 | 0.9 | 0.4 |
| H-clinoptilolite | 3.6 | 2.1 | 1.2 | 0.8 |
| H-erionite | 3.9 | 1.9 | 1.5 | 1.0 |

A comparison of H-erionite with SK-500 in ethylene amination was made using an ammonia to ethylene ratio of 4:1, a space velocity of 1000 hr$^{-1}$, and a temperature of 365°C. With SK-500, at the end of 20 days operation, propylene was observed and conversion had dropped from about 11 to about 8% by weight. On the other hand, after 42 days service the H-erionite catalyst was yielding 11% conversion. The calculated deactivation rate for SK-500 was 0.234%/day and for H-erionite it was less than 0.07%/day.

Although not intending to be bound by theory, another explanation for the enhanced conversion, selectivity and catalyst life at high temperatures is as follows:

The sorption cavities in erionite constitutes a three dimensional net work of pores. Each cavity has a length of 15.1 A and a free cross-sectional diameter of 6.3 to 6.6A. Reactants and products can enter or leave the cavity through six elliptical openings each has a minimum and maximum diameter of 3.5 and 5.2A respectively. These pores are large enough to allow uptake and egress of $C_2H_4$ (and other linear olefins) but too small to allow the formation of branched or cyclic hydrocarbons. The same analogy can be made for clinoptilolite. The free cross-section of these materials is different from A, X, or Y zeolites. The latter have spherical type cages with diameters of about 11—13A. This would allow the formation of large molecules (branched and cyclic) localized or strongly chemisorbed organic polymer of low mobility inside the pores which would result in blocking or obliteration of active sites. This of course would permit the formation of unsaturated and higher molecular weight organics and reduce conversion. The use of zeolites with a smaller pore system (erionite or clinoptilolite) decreases the change of ethylene polymerization, isomerization, and cyclodehydrogenation and eventually "coke" formation. This results in a much longer catalyst life compared to those of the large pore zeolites.

## Claims

1. In a vapor phase catalytic amination process for the production of amines from a reaction mixture comprising ethylene and a nitrogen compound consisting of ammonia at a temperature and pressure sufficient for effecting formation of said amine characterised by the improvement which comprises: employing as a catalyst for said process an alumino-silicate selected from the group of zeolites consisting of erionite, clinoptilolite and mixtures thereof.

2. The process of Claim 1 wherein said alumino-silicate is acidic.

3. The process of Claim 2 wherein said alumino silicate is rare earth or hydrogen ion exchanged.

4. The process of Claim 3 wherein the pressure in said amination is from $4.1 \times 10^6$—$7.6 \times 10^6$Pa (600—1100 psig), and the gas hourly space velocity is from 500—5,000.

5. The process of Claim 4 wherein the temperature in said amination is from 300—400°C.

6. The process of Claim 5 wherein the mole feed ratio of ammonia to ethylene is from 1—10:1.

7. A method for making amines which comprises contacting ethylene and ammonia in the vapor phase with a catalyst and an acidic alumino-silicate selected from the group consisting of erionite and clinoptilolite or mixtures thereof under amination conditions which include a temperature in the range of 300—400°C, a pressure in the range of $4.1 \times 10^6$—$7.6 \times 10^6$Pa (600—1100 psig), a mole ratio of ammonia to olefin of 1—10:1 and a gas hourly space velocity of 500—5000.

8. The process of Claim 7 wherein said acidity is provided by trivalent rare earth metal or hydrogen ions.

## Patentansprüche

1. Katalytisches Aminierungsverfahren in der Dampfphase zur Herstellung von Aminen aus einem Reaktionsgemisch, welches Ethylen und eine Stickstoffverbindung enthält, bestehend aus Ammoniak bei einer Temperatur und einem Druck die hinreichend sind, um die Bildung des Amins zu bewirken, dadurch gekennzeichnet, daß als ein Katalysator für das Verfahren ein Aluminosilikat eingesetzt wird, welches aus der Gruppe von Zeolithen, bestehend aus Erionit, Clinoptilolit und Gemischen davon, ausgewählt ist.

2. Verfahren nach Anspruch 1, worin das Aluminosilikat sauer ist.

3. Verfahren nach Anspruch 2, worin das Alumosilikat mit einem Seltenerd- oder Wasserstoffion ausgetauscht ist.

4. Verfahren nach Anspruch 3, worin der Druck bei der Aminierung 4,1×10⁶ bis 7,6×10⁶Pa (600 bis 1100 psig) beträgt und die stündliche Raumgeschwindigkeit des Gases 500 bis 5000 beträgt.

5. Verfahren nach Anspruch 4, worin die Temperatur bei der Aminierung 300 bis 400°C beträgt.

6. Verfahren nach Anspruch 5, worin das molare Einspeisungsverhältnis von Ammoniak zu Ethylen 1 bis 10:1 beträgt.

7. Verfahren zur Herstellung von Aminen, wobei Ethylen und Ammoniak in der Dampfphase mit einem Katalysator und einem sauren Alumosilikat, welches aus der Gruppe von Erionit und Clinoptilolit oder Gemischen davon ausgewählt ist, unter Aminierungsbedingungen, die eine Temperatur im Bereich von 300 bis 400°C, einen Druck im Bereich von 4,1×10⁶ bis 7,6×10⁶Pa (600 bis 1100 psig), ein Molverhältnis von Ammoniak zu Olefin von 1 bis 10:1 und eine stündliche Raumgeschwindigkeit des Gases von 500 bis 5000 umfassen, in Berührung gebracht werden.

8. Verfahren nach Anspruch 7, worin die Acidität durch trivalente Seltenerdmetall- oder Wasserstoffionen geschaffen wird.

**Revendications**

1. Procédé amélioré d'amination catalytique en phase vapeur pour la production d'amines à partir d'un mélange réactionnel comprenant de l'éthylène et un composé azoté consistant en ammoniaque, à une température et à une pression suffisantes pour effectuer la formation de l'amine, caractérisé en ce qu'on utilise comme catalyseur pour ce procédé un alumino-silicate choisi parmi le groupe des zéolites consistant en érionite, clinoptilolite et leurs mélanges.

2. Procédé suivant la revendication 1, caractérisé en ce que l'alumino-silicate est acide.

3. Procédé suivant la revendication 2, caractérisé en ce que l'alumino-silicate a été soumis à échange d'ion avec une terre rare ou l'hydrogène.

4. Procédé suivant la revendication 3, caractérisé en ce que la pression de l'amination est de 4,1×10⁶—7,6×10⁶Pa (600—1100 psig), et la vitesse spatiale horaire du gaz est de 500—5.000.

5. Procédé suivant la revendication 4, caractérisé en ce que la température de l'amination est de 300—400°C.

6. Procédé suivant la revendication 5, caractérisé en ce que le rapport d'alimentation molaire entre l'ammoniaque et l'éthylène est de 1—10:1.

7. Procédé de fabrication d'amines caractérisé en ce qu'on met en contact de l'éthylène et de l'ammoniaque en phase vapeur avec un catalyseur et un alumino-silicate acide choisi parmi le groupe consistant en érionite et clinoptilolite ou leurs mélanges, sous des conditions d'amination comprenant une température dans la gamme de 300—400°C, une pression dans la gamme de 4,1×10⁶—7,6×10⁶Pa (600—1100 psig), un rapport molaire entre l'ammoniaque et l'oléfine de 1:10:1 et une vitesse spatiale horaire du gaz de 500—5.000.

8. Procédé suivant la revendication 7, caractérisé en ce que l'acidité est fournie par des ions de métal trivalent des terres rares ou d'hydrogène.